# EUROPEAN PATENT APPLICATION

(11) **EP 3 085 249 A1**
(43) Date of publication of application: **26.10.2016**
(21) Application number: 16166947.8
(22) Date of filing: 25.04.2016
(51) Int. Cl.: A23L 33/00, A23L 2/66, A61K 38/01, A61K 35/20, A61K 36/48, A23L 33/185, A23L 33/19

(54) **A HIGH PROTEIN NUTRITIONAL COMPOSITION**

(30) Priority: 24.04.2015 IE 20150120
(71) Applicant: Nualtra Ltd, Limerick (IE)
(72) Inventor: GOUGH, PAUL, County Limerick (IE)
(74) Representative: Litton, Rory Francis

(57) **Abstract**

Provided herein are nutritional compositions that have a high content in proteins and a high caloric density. Also provided are related methods for formulating the nutritional compositions according to the invention. The present invention advantageously provides a heat-sterilized liquid nutritional composition with high energy content, designed to meet the nutritional needs of persons in need thereof, in particular malnourished patients, elderly and patients with certain disease conditions. The composition provides an increased amount of energy per unit volume while providing a good palatable liquid to allow the composition to be easily consumed orally. In addition, the taste of the composition is not diminished.

## Description

The present invention relates to the field of functional foods, more particularly to the field of Oral Nutritional Supplements (ONS). The present invention relates more particularly to a new and useful nutritional composition.

Malnutrition is a common health problem both in Ireland and the UK. A nutrition screening survey carried out in 2011 in collaboration with the Irish Nutrition and Dietetic Institute concluded that 27% to 33% of people who are admitted to hospital in Ireland are found to be malnourished leading to 30% longer hospital stays. Malnutrition is the condition that develops when the body does not get the right amount of the vitamins, minerals, and other nutrients it needs to maintain healthy tissues and organ function. Malnutrition can occur in people who are either undernourished or overnourished. Undernutrition is a consequence of consuming too few essential nutrients or using or excreting them more rapidly than they can be replaced. Infants, young children, and teenagers need additional nutrients. So do women who are pregnant or breastfeeding. Nutrient loss can be accelerated by diarrhoea, excessive sweating, heavy bleeding (haemorrhage), or kidney failure. Nutrient intake can be restricted by age-related illnesses and conditions, excessive dieting, food allergies, severe injury, serious illness, a lengthy hospitalization, or substance abuse. Overnutrition results from eating too much, if the diet does not contain the right balance of nutrients, not exercising enough, or taking too many vitamins or other dietary replacements.

Malnutrition begins with changes in nutrient levels in blood and tissues. Alterations in enzyme levels, tissue abnormalities, and organ malfunction may be followed by illness and death. This condition can affect growth, physical health, mood, the senses, behaviour and many of the functions of the body. High risk groups include: older people over the age of 65, particularly if they are staying in a care home or nursing home; people with long-term conditions such as diabetes or kidney disease; people with cancer; people who abuse drugs and/or alcohol; and people on low incomes. Malnutrition is both a cause and consequence of disease, delaying recovery from illness and increasing the risk of complications. This is at enormous cost to individuals, health care systems and society.

A treatment option for a patient suffering from malnutrition but who is able to eat normally is to provide food that has an extra nutrient content, such as high-protein/high calorie snacks or to consider oral nutritional supplementation (ONS). ONS are typically ready-made, multinutrient liquid supplements that are energy dense and contain both macronutrients (protein, carbohydrate and fat) and micronutrients (vitamins, minerals and trace elements) and may also contain fibre.

Various commercial sources of carbohydrates exist and may be utilized in liquid nutritional compositions. Examples of suitable carbohydrates or sources thereof for use in the liquid nutritional compositions include, but are not limited to, maltodextrin, native, hydrolyzed or modified starch or cornstarch, isomaltulose, glucose polymers, corn syrup, corn syrup solids, rice-derived carbohydrates, glucose, fructose, lactose, sucrose, high fructose corn syrup, honey, sugar alcohols (e.g., maltitol, erythritol, sorbitol), slow digesting starches, barley beta glucan, glycerin, sucromalt, inulin and combinations thereof. It will be readily appreciated that the above list of carbohydrate sources is not exhaustive and the carbohydrate source in a liquid nutritional composition may be readily substituted.

Various commercial sources of fat exist and may be utilized in nutritional compositions. Examples of suitable fats or sources thereof for use in the nutritional compositions disclosed herein, include, but are not limited to, coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, fish oil, cottonseed oils, lecithin, datem, mono and di glycerides, milk phospholipids, polyglycerol esters, citric acid esters and combinations thereof. It will be readily appreciated that the above list of fat sources is not exhaustive and the fat source in a liquid nutritional composition may be readily substituted.

Oral nutritional supplements may also additionally contain fibre, some of which may be partially soluble or a combination of soluble and insoluble fibres may be used. Examples of suitable fibres, or sources thereof, include, but are not limited to, soy polysaccharides, resistant starch, inulin, cellulose and oligofructose. It will be readily appreciated that the above list of fibre sources is not exhaustive and the fibre source in a liquid nutritional composition may be readily substituted.

A variety of nutritional supplements are available, including nutritionally complete, nutritionally incomplete (e.g. fat-free or lacking some essential micronutrients) and modular (e.g. usually containing one or two energy sources only) varieties. Problems associated with the use of oral nutritional supplements may include:
- Reduction in the intake of normal food - when the use is generally intended to supplement normal eating and drinking,
- Low palatability - some supplements may not be suitable for some people who may not like the taste and texture,
- Adverse effects - usage of these products can cause adverse effects such as nausea and diarrhoea,
- Wastage - it is estimated that up to a third of all product dispensed is wasted.

These problems affect the patient's compliance with the treatment. The term "compliance'" refers to the extent to which a person's behaviour coincides with medical advice. With respect to drug therapy, compliance is defined as the degree of correspondence of the actual dosing history with the prescribed drug regimen. Non-compliance is a major obstacle to the effective delivery of health care.

Protein, in particular, is a necessary part of the diet. Proteins are useful for building muscle, for forming hormones and enzymes, and for providing scaffolding within and between cells. They also provide an energy source, as well as the amino acids useful for general cell growth and repair. Some individuals have a greater need for protein in their diets, such as individuals who are malnourished, or diseased, children who need protein to aid in growth, and elderly individuals who may lose muscle mass during the aging process. Because ONS may provide a variety of nutrients in an easy-to-administer form, efforts have been made to add protein of various kinds to beverages to increase their nutritional value.

However a real problem with incorporating proteins in drinks is the inability of proteins to be dissolved in acidic media. Additionally their structure can be damaged by the sterilization process, therefore affecting their nutritive value and digestibility and also the shelf life of the product containing said proteins.

A solution to these above problems is to include milk proteins. The term "Skimmed milk" refers to milk which has a fat content of from 0 to 0.5% w/w and an average fat content of 0.1% w/w. Skimmed milk therefore has nearly all the fat removed.

Milk contains approximately 3.5% w/w of protein, which can be divided into two main groups: casein proteins and whey proteins. Approximately 80% w/w of the protein in cow milk is casein based with the remaining 20% w/w is whey based. "Casein" is the predominant protein in milk and can be divided into four major types: alpha, beta, gamma and kappa caseins. "Whey" protein refers to the rest of the milk protein and is composed predominantly of beta -lactoglobulin and alpha-lactalbumin but also include serum albumin, immunoglobulins (IgA, IgG, IgM), proteases, peptones, lactoferrins and transferrins.

The term "micellar milk protein" refers to milk protein which is native protein; the term likewise refers to milk protein which has not been denatured. While in its non-denatured state, proteins in milk naturally form micelles in an aqueous environment. Denaturing results in a breakdown of a portion of these naturally occurring protein micelles present in milk.

Micellar casein, also named native micellar casein, is a high quality milk protein and naturally occurring in milk at a concentration of about 2.6 g/100mL. It is concentrated by a process that does not, or does not substantially, cause the casein proteins to denature and it is marketed as Micellar Casein Isolate (MCI). Fresh skimmed milk is subjected to a filtration process, in much the same process used to concentrate whey protein, to produce a pure milk protein with its native structure intact, the pure milk protein being substantially free from denatured protein. The resulting material contains more than 95 % w/w micellar casein, the rest mainly being whey protein, other non-protein nitrogen and other constituents, such as lactose. MCI has an intrinsic low viscosity and a liquid composition comprising said MCI is therefore considered easy to ingest and consume.

The term "milk protein concentrate" (MPC) is generally used to refer to a milk protein containing product that has had a considerable amount of the inherent water from ordinary milk removed and also has had inherent fat from the ordinary milk removed. As used herein, the phrase milk protein concentrate should be understood to mean a source of milk protein that comprises milk that has had a considerable amount of water and a portion of the inherent fat removed. Generally, commercially available milk protein concentrates contains less than 85% w/w of protein, minimal fat (i.e., 1 to 3% w/w), and lactose (at >2% w/w).

The term "milk protein isolate" (MPI) is generally used to refer to a type of milk-protein containing product that has not only had a considerable amount of the inherent water from ordinary milk removed and inherent fat but also a certain amount of inherent lactose removed. In most instances, milk protein isolates can be considered to be a type of further purified milk protein concentrate. Generally, commercially available milk protein isolates contain about 85 to 90 % w/w of protein (or more), about 2 to 5 % w/w of lactose, minimal fat (i.e., 1 to 3% w/w), lactose (at about 1 to 2% w/w) and about 5 to 6 % w/w of water. An MPI generally has a higher protein content (anywhere from 3.5% to 6% higher than MPCs) because it contains much less lactose than an MPC.

The term "whey milk protein" refers to milk protein which is native protein, the major whey proteins in cow milk are ß-lactoglobulin and a-lactalbumin. Other whey proteins include immunoglobulins and serum albumin. Whey proteins also include a long list of enzymes, hormones, growth factors, nutrient transporters, disease resistance factors, and others. Whey is a fast-digesting protein while casein is a slow-digesting protein. Whey proteins also have higher levels of leucine, a potent amino acid that stimulates protein synthesis. Whey protein is superior at augmenting protein synthesis rapidly, but this positive effect is short-lived. Consuming repeated doses of whey allows for sustained high levels of blood amino acids and repeated bursts of protein synthesis that provide superior effects on muscle protein balance. Common forms of whey protein include whey protein concentrate, whey protein isolate and partially hydrolyzed whey protein. Various commercial sources of milk protein exist, where whey protein makes up to as much as 75% w/w of the commercial milk protein source.

Sources of milk proteins above have a considerable amount of the inherent water content of milk removed, with MPIs having as little as 5 to 6 %w/w of water this is insufficient to provide the much needed minimum hydration requirements of patients suffering from insufficient nutrient intake, who often do not drink enough and consequently require higher than average hydration.

Combinations of micellar casein and/or caseinate proteins are well known for use as the protein source in nutritional compositions. For example, US 5,683,984 (Nestec S.A.) teach to replace all of the caseinate in a medium energy nutritional formulation (1 kcal/ml) by native micellar casein to obtain a formulation essentially containing native micellar casein with a low viscosity and a thermal stability to withstand sterilization. It discloses a composition containing a maximum of 7 vol% of native micellar casein. EP 2 230 940 B1 (Nutricia) discloses a heat-sterilized liquid enteral nutritional composition comprising 8 to 14 g of protein per 100 ml of the composition, said protein including micellar casein and caseinate, the composition having an energy density of at least 2.0 kcal/ml, wherein the weight ratio of micellar casein and caseinate ranges from 90:10 to 35:65, and the combined amount of micellar casein and caseinate is at least 70wt% of the total protein contained in the composition. Milk proteins are a convenient and low cost protein source. However, difficulties with incorporating these proteins in high quantity in ONS include poor shelf life and poor heat stability of the proteins. Furthermore milk proteins can exhibit digestibility and palatability problems for the patient. In addition, increasing calories and/or proteins in a nutritional liquid composition may increase the overall viscosity of the composition. Highly viscous nutritional compositions are often perceived as having an unpleasant mouthfeel and as generally undesirable. Furthermore, many protein variants have undesirable flavour characteristics. This can make the liquid nutritional composition difficult to consume or administer, and can also diminish the taste of the nutritional composition. Moreover organoleptic characteristics such as mouthfeel play an important role in the selection of liquid nutritional compositions by consumers. There is a technical difficulty in making the formulation of high caloric density liquid nutritional compositions in a fashion that is palatable to a wide variety of consumers. By "high caloric density" we refer to a high energy content provided in a small unit measure of food compared relatively to the energy content of other foods or compositions for the same unit measure. One way to express the energy content or calorie content of a liquid nutritional composition is on a kilocalorie per serving basis. Still further there is a practical problem in that the nutritional element must be relatively inexpensive to justify its inclusion, notably to be readily producible in high yield for a relatively low cost.

Combinations of animal and plant based proteins is also well known. For example, WO 02/098242 A1 (Nestlé) discloses a calorically dense liquid oral supplement (2.25 kcal/ml) based on a (60:40) soy protein isolate/caseinate mixture with a protein level of 9 g/100 ml, 12.25 g/100 ml of fat, and 19.7 g/100 ml of digestible carbohydrates.

The term "soy protein" refers to is a protein that is isolated from the soybean plant. It is made from soybean meal that has been dehulled and defatted. Soy protein may be provided by one or more than one source. Commercial sources of soy protein are well known and common forms of soy protein include soy protein concentrates and soy protein isolates. Soy offers a vegetarian source of protein that is cholesterol free and high in the amino acid glutamine, the latter of which is particularly good for runners and endurance athletes.

None of the prior art describes a composition comprising a combination of whey protein with soy proteins; furthermore the combination of casein, whey and soy is not known.

There is always a need to obtain alternative high protein beverage suitable as oral nutritional supplements. Therefore, a need exists for improved nutritional compositions, with an increased amount of protein and calories per unit volume for treating a person in need. In particular a need exists for a high protein composition where the protein source provides a combination of animal and plant proteins, and more particularly where the animal proteins comprise of both casein and whey milk proteins.

In view of the above, one of the objectives of this invention is to provide high protein nutritional composition, stable, attractive, which has a good organoleptic properties for providing nutrition, either as a supplement, or as a complete nutrition, with a high energy content, to a person, in particular to a malnourished person or a person in need of a supplement of proteins.

### Statements of Invention

Provided herein are nutritional compositions that have a high content in proteins and a high caloric density. Also provided are related methods for formulating the nutritional compositions according to the invention.

The present invention advantageously provides a heat-sterilized liquid nutritional composition with high energy content, designed to meet the nutritional needs of persons in need thereof, in particular malnourished patients, elderly and patients with certain disease conditions. The composition provides an increased amount of energy per unit volume while providing a good palatable liquid to allow the composition to be easily consumed orally. In addition, the taste of the composition is not diminished.

In an embodiment, high protein component includes one or more proteins, peptides, and amino acids, and precursors and metabolites of proteins, peptides, and amino acids in an amount that provides at least about 10% of the total calories of the nutritional composition, preferably at least 15%.

In an embodiment, the nutritional compositions provide at least 8 grams of protein per 100 mL of composition, preferably 8 to 12 grams of protein per 100 mL of composition.

The nutritional compositions according to invention can have varying calorie contents. In certain embodiments, the liquid nutritional compositions according to the invention have an amount of calories of 100 to 300 kilocalories per 100mL. In certain embodiments according to invention, the liquid nutritional compositions have a high calorie content of 200 to300 kilocalories (kcal) per 100mL, preferably 200 to280 kcal per 100mL, more preferably 220 to260kcal per 100mL. Most preferably, the nutritional compositions have a caloric density of 240 to 250 kcal per 100mL.

In certain embodiments, the nutritional compositions comprise micellar milk proteins, casein protein, whey milk protein, soy proteins and any combination of two or more thereof.

The nutritional compositions according to the invention are designed to provide a high amount of energy and to provide a high amount of easily digestible proteins. Moreover the proteins have an animal and vegetal origin, therefore providing all the essential aminoacids. Advantageously, the combination of micellar and non-micellar milk proteins with soy protein allows for improved organoleptic qualities and the production of liquid nutritional compositions displaying low viscosity along with a high caloric density. Furthermore soy protein provides an added benefit of cholesterol lowering properties.

Preferably, when whey protein is utilized in the liquid nutritional compositions disclosed herein, the amount of whey protein utilized in the liquid nutritional compositions is generally up to 25 % w/w (i.e., 0 to 25 % w/w) of the total protein content present in the overall composition. In certain embodiments 1 to25 % w/w of the total protein content of the overall composition, preferably 1 to 15 % w/w of the total protein of the overall composition, and more preferably 1 to10 % w/w of the total protein content of the overall composition. In certain embodiments, where both whey protein and soy protein are used in the liquid nutritional compositions, the total combined amount of soy and whey protein used comprises 1 to25 % w/w of the total protein content of the overall composition.

Preferably, milk protein isolate and skimmed concentrated milk are used in the nutritional composition according to the present invention as a source of micellar casein and whey proteins.

Preferably, when soy protein is utilized in the nutritional compositions disclosed herein, the amount of soy protein that may be utilized in the nutritional compositions is generally up to 25 % w/w (i.e., 0 to 25 % w/w) of the total protein content of the overall composition. In certain embodiments 1 to 25 % w/w of the total protein content of the overall composition, preferably 1 to15 % w/w of the total protein content of the overall composition, and more preferably 1 to10 % w/w of the total protein in yet other embodiments. Preferably, the soy protein is a soy protein isolate.

In a preferred embodiment, the proteins comprise micellar casein and whey, casein and whey and soy proteins.

Advantageously, the particular mixture of micellar casein and whey, casein and whey, and soy protein in this embodiment solves the problems of shelf stability and palatability of the nutritional composition. The nutritional compositions according to the invention have a relatively high protein concentration (i.e. 8 to12 grams per 100mL of nutritional composition), while retaining a shelf stability generally only achieved by nutritional compositions having lower protein concentrations.

Advantageously, the nutritional compositions according to the invention provide a highly nutritive amount of protein. Indeed casein and whey are two milk proteins both excellent sources of all the essential amino acids. Additionally, the nutritional compositions according to the invention provide a highly assimilable source of protein. Indeed whey and casein are better assimilated by the body when combined together. Since whey rapidly increases protein synthesis and casein blocks protein breakdown, a combination of both provides an optimal source of protein and a two pronged approach to combating malnutrition or malnutrition related conditions.

Moreover, the nutritional compositions according to the invention comprise soy protein.

In certain embodiments, carbohydrates are present in the nutritional composition according to the present invention. The carbohydrates may be provided by a single source or by more than one source. The particular amount of carbohydrate present in the liquid nutritional compositions may vary depending upon the desired amount of calories in the nutritional compositions. Carbohydrate content of the overall composition falls within the range of 20% w/v to 40% w/v, and preferably 20% w/v to 30% w/v. The amount of carbohydrate present in the liquid nutritional composition according to the present invention can also be characterized as a percentage of total calories of the overall composition and may vary widely from 30 to60%. In certain embodiments according to the invention, the carbohydrates comprise 30 to60%, preferably 35 to60%, and more preferably 38 to58% of the total calories of the overall liquid nutritional composition.

In certain embodiments, the nutritional composition of the present invention comprises fat or at least one source of fat. In an embodiment, the amount of fat according to certain embodiments is from 8 to 12 grams per 100mL, and preferably from 9 to 10 grams per 100mL of the overall liquid nutritional composition. Preferably, the amount of fat is from 30 to 50% of the total calories in the nutritional composition, including 30 to 45%, and 35 to 45% of the total calories in the nutritional composition.

For example, the nutritional composition of the present invention may comprise a lipid source with an n6:n3 fatty acid ratio in the range of 2:1 to 3:1.

The nutritional composition of the present invention may comprise water from 70 to 73% v/v of the overall composition. Advantageously water provides the minimum hydration requirements for patients with insufficient nutrient intake who often do not drink enough.

According to a preferred embodiment of the invention, there is provided a high protein composition comprising from 8 to 12 grams of protein per 100mL; from 8 to 12 grams of fat per 100mL; from 23 to 34 grams of carbohydrate per 100mL, characterised in that, the protein fraction comprises two or more of micellar casein and whey, casein and whey protein and soy protein or any combination thereof.

In certain embodiments, the nutritional compositions may contain other ingredients, examples of which include, but are not limited to, preservatives, antioxidants, emulsifying agents, buffers, pharmaceutical actives, additional nutrients, colorants, flavours, thickening agents and stabilizers. In certain embodiments, stabilizers present in the disclose embodiments include: octenyl succinic anhydride, gellan gum, alginate, pectin, guar gum, locust bean gum, konjack, carboxymethyl cellulose and microcrystalline cellulose and combinations thereof. Those of skill in the art will recognize that many different gum forms, in addition to those listed above, may be used in the liquid nutritional compositions disclosed herein and still fall within the disclosed embodiments.

In certain embodiments, the nutritional compositions may contain vitamins or related nutrients, examples of which include, but are not limited to, vitamin A, vitamin E, vitamin K, thiamine, riboflavin, pyridoxine, vitamin B12, carotenoids (e.g., lutein), niacin, folic acid, pantothenic acid, biotin, vitamin C, choline, inositol, salts, and derivatives thereof, and combinations thereof.

In certain embodiments according to the invention, the nutritional compositions contain minerals, examples of which include, but are not limited to, calcium, phosphorus, magnesium, iron, zinc, manganese, copper, sodium, potassium, molybdenum, chromium, selenium, chloride, and combinations thereof.

In certain embodiments according to the invention, the nutritional compositions also optionally include one or more masking agents to reduce or otherwise obscure the development of any residual bitter flavours and after taste in the emulsions over time. Suitable masking agents include natural and artificial sweeteners, sodium sources such as sodium chloride, and hydrocolloids, such as guar gum, xanthan gum, carrageenan, and combinations thereof. The amount of masking agent in the nutritional emulsion may vary depending upon the particular masking agent selected, other ingredients in the formulation, and other formulation or product target variables. Such amounts, however, most typically range from 0.1 % w/v to 3 % w/v, preferably from 0.15% w/v to 3 % w/v, and more preferably from 0.18% w/v to 2.5% w/v, of the overall nutritional emulsion.

In a preferred embodiment, the nutritional composition comprises at least one emulsifier selected from the group consisting of: lecithin, monoglycerides, diglycerides, polyglycerol esters, milk phospholipids, citric acid esters, and emulsifiers with a hydrophobic-lipophilic balance between 5 and 16.

In an embodiment, the nutritional composition comprises a chelating agent selected from the group consisting of divalent citrates, divalent phosphates, and combinations thereof. The chelating agent is present in an amount of 0.3% w/v to 0.5 % w/v.

In a preferred embodiment, the nutritional composition is formulated in a total volume of 150mL, or less, and preferably 125mL. Advantageously this volume allows a good source of nutrients and calories for a person in need. Additionally, this volume is enough to bring a nutritional supplement but also small enough to be well accepted by patients, therefore over 95% of the patients comply with the treatment.

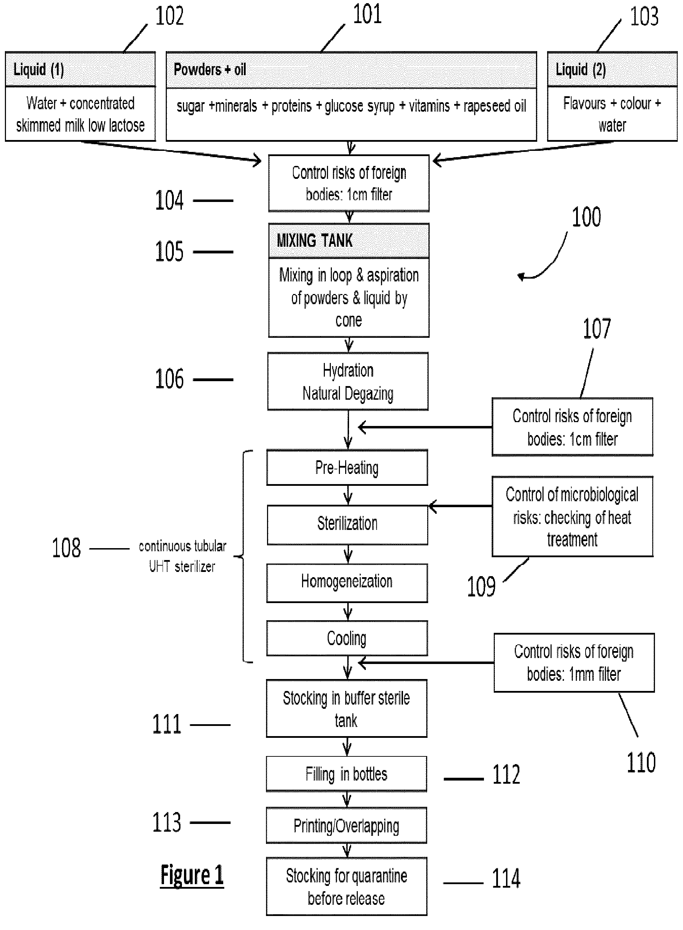

In a second aspect, the present invention concerns the use of a mixture of micellar casein and whey, casein and whey proteins, and soy proteins in the manufacture of a nutritional composition according to the present invention for providing nutrition to a person. Compositions comprising liquid micellar milk proteins, milk protein isolates are prepared separately, before being filtered, mixed together, hydrated and degazed, sterilized and packaged (example 9; figure 1).

In a third aspect, a method for the manufacture of a stable, high caloric density nutritional composition according to the invention is provided generally comprising the steps: filtration, mixing, hydration and degazing, sterilization and packaging.

In a fourth aspect, the present invention concerns a method of providing nutrition to a person in need thereof, comprising the steps of administering to said person the nutritional composition according to the present invention.

The present invention is an nutritional composition to be administered to patients in need thereof having a caloric density in the range of 0.9-1.6 kcal/ml, an osmolality in the range of 380-420 mOsm/kg water, and comprising a protein source accounting for about 12-20% of the calories of the composition, a carbohydrate source accounting for about 35-58% of the calories of the composition, a lipid source accounting for about 30-45% of the calories of the composition and probiotic micro-organisms.

The nutritional composition according to the present invention has a pH between 6 and 7.8, preferably between 6.3 and 7.3.

### Detailed Description of the Invention

The invention will be more clearly understood by the following description of some embodiments thereof, given by way of example only, with reference to the accompanying drawings, in which:
Figure 1 is a process flow diagram detailing the steps involved in making the composition in accordance with the present invention.

The process of making the nutritional composition, generally referred to by reference numeral 100, generally comprises preparation of a first mixture 101, a second mixture 102 and a third mixture 103; combining said mixtures together before filtration 104, using a filter of pore size ~1cm in size, mixing in a mixing tank 105 using a loop and aspiration of powders and liquid by cone; hydrating and degazing 106; filtration 107, using a filter of pore size ~1mm in size; sterilising 108, comprising a preheating step, a sterilisation step, a homogenisation step and a cooling step; during the sterilisation step 108, the combined mixture undergoes a microbiological test 109 to determine that sterilisation has been achieved; the combined mixture then undergoes a further filtration step 110 using a filter of pore size ~1mm in size, before being stocked in a buffer sterile tank 111 and incubated for 5 days at 53°C, in preparation for packaging, which comprises filling 112 under a protective atmosphere into bottles that have been pre-incubated at 30°C for 72hours with a D-count of PE= 0.5 to 1 g, and labelling 114 comprising printing and overwrapping. The finished product is then stocked for quarantine prior to sale 114 for about 12 to 18 months at 4 to 25°C, under relative humidity conditions from 30 to 75% R.H.

### Example 1: Macronutrient ingredients

Provided is the quantitative content of macronutrient ingredients (Table 1) in the overall nutritional composition according to the present invention. Macronutrients generally include proteins, fats and carbohydrates.

**Table 1: Macronutrient content of the composition**

| Macronutrient | Content level (g/100 mL of finished product) |
|---|---|
| Proteins | 9.6 |
| Fat | 9.6 |
| Carbohydrates | 28.8 |

### Example 2: Micronutrient ingredients

Provided is the quantitative content of micronutrients such as vitamins and minerals and trace elements (Table 2) as in the overall nutritional composition according to the present invention.

**Table 2: Vitamin and Mineral inqredient listing**

| Vitamins | | |
|---|---|---|
| | Dimension | Nominal value |
| Vit A (Retinol acetate) | µgRE/100ml | 200 |
| Vit E (DL alpha tocopherol acetate) | mgTE/100ml | 2.8 |
| Vit C (L-ascorbic acid) | mg/100ml | 24 |
| Vit B1 (Thiamin mononitrate) | mg/100ml | 0.5 |
| Vit B2 (Riboflavin) | mg/100ml | 0.5 |
| Niacine (nicotinamide) | | 4.8 |
| Vit B5 (Calcium D pantothenate) | mg/100ml | 1.6 |
| Vit B6 (pyridoxine HCl) | mg/100ml | 0.6 |
| Biotin (D-Biotin) | µg/100ml | 9.6 |
| Folic acid (pteroylmonoglutamic acid) | µg/100ml | 68 |
| Vit B12 (Cyanocobalamin) | µg/100ml | 0.8 |
| Vit D3 (cholecalciferol) | µg/100ml | 2 |
| Vitamin K (phytomenadione) | µg/100ml | 12 |
| Minerals and Trace Elements | | |

| | Dimension | Nominal Value |
|---|---|---|
| Calcium | mg/100ml | 168 |
| Copper | µg/100ml | 410 |
| Iron | mg/100ml | 3.2 |
| Potassium | mg/100ml | 240 |
| Magnesium | mg/100ml | 32 |
| Manganese | mg/100ml | 0.8 |
| Sodium | mg/100ml | 101 |
| Phosphorus | mg/100ml | 160 |
| Zinc | mg/100ml | 2.5 |
| Chromium | µg/100ml | 16 |
| Chloride | mg/100ml | 89 |
| Fluor | mg/100ml | 0.2 |
| Molybdenum | µg/100ml | 20 |
| Iodine | µg/100ml | 30 |
| Selenium | µg/100ml | 14 |
| Choline | mg/100ml | 60 |

### Example 3: Properties of the nutritional composition

Analysis demonstrates that the nutritional composition according to the present invention has desirable viscosity and pH (table 3), as well as providing an energy dense composition with desirable osmolarity ranging from 901.6 to 966mOsm/L. Further nutrient profile is also herein provided where, among other nutrient ingredients mono and disaccharides range from 7.9 to 8.3 g/100ml; polysaccharides range from 20.5 to 20.9 g/100ml; and, sacharose ranges from 3.5 to 4.05.

**Table 3: volume viscosity and pH.**

| | | |
|---|---|---|
| EST/Dry Matter | g/100ml | 49.6 |
| Mass Volume / Density | g/L | 1140 |
| pH | | 6.70 |

**Table 4: Kilocalories, osmolarity and main nutrient content.**

| Main Nutrients | | | |
|---|---|---|---|
| | Dimension | Normal Value | Origin of data |
| Energy | Kcal/100ml | 240 | calculation |
| | KJ/100ml | 1008 | |
| Osmolarity | MOsm/l | 966 | calculation |
| | MOsm/l | 901.6 | |
| Saturated fatty acid | g/100ml | 0.92 | calculation |
| Monosaturated fatty acid | g/100ml | 5.96 | calculation |
| Polysaturated fatty | g/100ml | 2.73 | calculation |
| Linoleic acid | g/100ml | 1.87 | calculation |
| W6/W3 | g/100ml | 2.22 | calculation |
| Mono & disaccharides | g/100ml | 7.9 | calculation |
| | | 8.2 | |
| | | 8.3 | |
| Polysaccharides | g/100ml | 20.9 | calculation |
| | | 20.6 | |
| | | 20.5 | |
| Saccharose | g/100ml | 3.6 | calculation |
| | | 4.05 | |
| Lactose | g/100ml | <0.5 | calculation |

### Example 4: Typical nutritional compositions

Provided herein are examples of nutritional beverages and typical ingredient listings (Table 1) for the same having from 8 to 12 grams of protein per 100mL, from 8 to 12 grams of fat per 100mL, and from 23 to 34 grams of carbohydrates per 100mL and a neutral to basic pH of a value from 6.8 to 7.3.

**Table 5: Chocolate-Hazelnut flavoured nutritional beverage**

| Ingredients | Composition (=> allergens) |
|---|---|
| Skimmed concentrated milk "low | Skimmed concentrated milk (=> milk, lactose) |
| Glucose syrup | Glucose syrup (Maize) |
| Tap water | Tap water |
| Rapeseed oil | Rapeseed oil |
| Milk protein | Milk protein, soya lecithin, calcium phosphate = technological auxiliary (=> milk, lactose, soya lecithin) |
| Sugar | Sugar beet |
| Isolated Soy protein | Isolated soya protein (=> soya, sulphite (<35ppm)) |
| Caramel colour E150b | Saccharose (beet), sodium sulphite; auxiliary: acid citric, sunflower oil, soda (=>sulphites (1020ppm)) |
| Magnesium (II) citrate | Magnesium citrate extra pur |
| Sodium hydrogeno phosphate E339 (ii) | Sodium phosphate |
| Tri potassium citrate E332 | Tri potassium citrate = sugar beet |
| Monoglycerides (E471) | E471 (palm oil) |
| Potassium Chloride E508 | Potassium chlorure |
| Chocolate Flavour E4140W | Flavouring preparation, flavouring substances, maltodextrine (wheat, potatoes) |
| Chocolate-Hazelnut flavour | Flavouring thermal process, flavouring substances, flavouring preparation, natural flavouring substances, propylene glycol E-1520 (81%), ascorbic acid 0.03% (=>milk, lactose) |
| F340 ii: K2HPO4 | K2HPO4 (E340 ii) |
| Choline Chloride | Choline chloride |
| Trisodium Citrate | Saccharose (cane and treacle of beet) |
| Potassium hydroxide E525 | Potassium hydroxide E525 |
| Ascorbic acid | Ascorbic acid (synthetic) |
| Vitamin mix GNE | Vitamin E, Niacin, Vitamin A, Panthothenic Acid, Biotin, Folic acid anhydrous, Vitamin B1, Vitamin B6, Vitamin D, Vitamin B2, Vitamin K1, Vitamin B12, maltodextrin is used as a carrier |
| Masking flavour | Substance identical to natural flavouring substance, propylene glycol (86.5%) |
| Ferric (III) Pyrophosphate | Ferric pyrophosphate (21 % Fe) |
| Zinc (II) sulfate.7H20 | Zinc sulfate |
| Sodium selenite (IV) 1% | Sodium selenite, maltodextrin is used as a carrier |
| Maganese (II) sulfate | Manganese sulfate |
| Copper (II) sulphate | Copper sulphate |
| Sodium fluoride | Sodium fluoride |
| Chromium (III) chloride | Chromium chloride |
| Sodium molybdate | Sodium molybdate |
| Potassium iodide | Potassium iodide |

**Table 6: Vanilla flavoured nutritional beverage**

| Ingredients | Composition (=> allergens) |
|---|---|
| Skimmed concentrated milk "low | Skimmed concentrated milk (=> milk, lactose) |
| Glucose syrup | Glucose syrup (Maize) |
| Tap water | Tap water |
| Rapeseed oil | Rapeseed oil |
| Milk protein | Milk protein, soya lecithin, calcium phosphate = technological auxiliary (=> milk, lactose, soya lecithin) |
| Sugar | Sugar beet |
| Isolated Soy protein | Isolated soya protein (=> soya, sulphite (<35ppm)) |
| Magnesium (II) citrate | Magnesium citrate extra pur |
| E339 (II):Sodium hydrogeno phosphate | Sodium phosphate |
| E332: Tri potassium citrate | Tri potassium citrate = sugar beet |
| E471: emulsifier: Mono & diglycerides of fatty acids | E471 (palm oil) |
| E508: Potassium Chloride | Potassium chlorure 99%, magnesium hydroxide carbonate 1% |
| E340 II: K2HPO4 | K2HPO4 (E340 II) |
| Choline Chloride | Choline chloride |
| Vanilla Flavour | propylene glycol (82%), flavouring substances |
| E331 (III): Trisodium Citrate | Trisodium Citrate E331 (III) |
| Vitamin C (Ascorbic acid) | Ascorbic acid (synthetic) |
| E160 a (II): Natural β-Carotene | Maltodextrin, acacia gum E414, Vegetable oil, (coconut (MCT oil, sunflower oil), sorbitol E420 (<5%), natural Beta Carotene E 160a (II), ascorbic acid E330, DL alpha tocopherol E307 (<1%) |
| Ferric (III) Pyrophosphate | Ferric pyrophosphate (21% Fe) |
| Mix Vit NJTRIPLEN V2 | Vitamin E, Niacin, Vitamin A, Panthothenic Acid, Biotin, Folic acid anhydrous, Vitamin B1, Vitamin B6, Vitamin D, Vitamin B2, Vitamin K1, Vitamin B12, maltodextrin is used as a carrier |
| Zinc (II) sulfate.7H20 | Zinc sulfate |
| Sodium selenite (IV) 1% | Sodium selenite, maltodextrin is used as a carrier |
| Maganese (II) sulfate | Manganese sulfate |
| Copper (II) sulphate | Copper sulphate |
| Sodium fluoride | Sodium fluoride |
| Chromium (III) chloride | Chromium chloride |
| Sodium molybdate | Sodium molybdate |
| Potassium iodide | Potassium iodide |

**Table 7: Strawberry flavoured nutritional beverage**

| Ingredients | Composition (=> allergens) |
|---|---|
| Skimmed concentrated milk "low | Skimmed concentrated milk (=> milk, lactose) |
| Glucose syrup | Glucose syrup (Maize) |
| Tap water | Tap water |
| Rapeseed oil | Rapeseed oil |
| Milk protein | Milk protein, soya lecithin, calcium phosphate = technological auxiliary (=> milk, lactose, soya lecithin) |
| Sugar | Sugar beet |
| Isolated Soy protein | Isolated soya protein (=> soya, sulphite (<35ppm)) |
| Magnesium (II) citrate | Magnesium citrate extra pur |
| E339 (II):Sodium hydrogeno phosphate | Sodium phosphate |
| E332: Tri potassium citrate | Tri potassium citrate = sugar beet |
| E471: emulsifier: Mono & diglycerides of fatty acids | E471 (palm oil) |
| E508: Potassium Chloride | Potassium chlorure 99%, magnesium hydroxide carbonate 1% |
| E340 II: K2HPO4 | K2HPO4 (E340 II) |
| Choline Chloride | Choline chloride |
| Strawberry Flavour | flavouring substances (5-10%), natural flavouring substances (0.1-1%), propylene glycol E1520 (60%), ethyl alcohol (15-20%), water (10-15%), acetic acid E260 (0.6%), tocopherol alpha (E307 6ppm) |
| E331 (III): Trisodium Citrate | Trisodium Citrate E331 (III) |
| Vitamin C (Ascorbic acid) | Ascorbic acid (synthetic) |
| E120:Carmin colour | Colour cochineal E120 (1.6%) (Dactyoplus coccus costa), sodium carbonate (E500), water maltodextrin (potatoes) |
| Ferric (III) Pyrophosphate | Ferric pyrophosphate (21 % Fe) |
| Mix Vit NJTRIPLEN V2 | Vitamin E, Niacin, Vitamin A, Panthothenic Acid, Biotin, Folic acid anhydrous, Vitamin B1, Vitamin B6, Vitamin D, Vitamin B2, Vitamin K1, Vitamin B12, maltodextrin is used as a carrier |
| Zinc (II) sulfate.7H20 | Zinc sulfate |
| Sodium selenite (IV) 1% | Sodium selenite, maltodextrin is used as a carrier |
| Maganese (II) sulfate | Manganese sulfate |
| Copper (II) sulphate | Copper sulphate |
| Sodium fluoride | Sodium fluoride |
| Chromium (III) chloride | Chromium chloride |
| Sodium molybdate | Sodium molybdate |
| Potassium iodide | Potassium iodide |

**Table 8: banana flavoured nutritional beverage**

| Ingredients | Composition (=> allergens) |
|---|---|
| Skimmed concentrated milk "low | Skimmed concentrated milk (=> milk, lactose) |
| Glucose syrup | Glucose syrup (Maize) |
| Tap water | Tap water |
| Rapeseed oil | Rapeseed oil |
| Milk protein | Milk protein, soya lecithin, calcium phosphate = technological auxiliary (=> milk, lactose, soya lecithin) |
| Sugar | Sugar beet |
| Isolated Soy protein | Isolated soya protein (=> soya, sulphite (<35ppm)) |
| Magnesium (II) citrate | Magnesium citrate extra pur |
| E339 (II):Sodium hydrogeno phosphate | Sodium phosphate |
| E332: Tri potassium citrate | Tri potassium citrate = sugar beet |
| E471: emulsifier: Mono & diglycerides of fatty acids | E471 (palm oil) |
| E508: Potassium Chloride | Potassium chlorure 99%, magnesium hydroxide carbonate 1% |
| E340 II: K2HPO4 | K2HPO4 (E340 II) |
| Choline Chloride | Choline chloride |
| Bannana Flavour | flavouring preparations, natural flavouring substances, propylene glycol E1520 (28%), alcohol (48.5%) |
| E331 (III): Trisodium citrate | Trisodium Citrate E331 (III) |
| E525: Potassium hydroxide | Potassium hydroxide E525 |
| Vitamin C (Ascorbic acid) | Ascorbic acid (synthetic) |
| E100:Natural Colour Curcumin | Flavouring preparations, E100 curcumin (8.8%), polysorbate E433 (75%) |
| Ferric (III) Pyrophosphate | Ferric pyrophosphate (21% Fe) |
| Masking Flavour (Flavour) | Flavouring preparation, natural flavouring substance, polypropylene glycol (86%) |
| Mix Vit NJTRIPLEN V2 | Vitamin E, Niacin, Vitamin A, Panthothenic Acid, Biotin, Folic acid anhydrous, Vitamin B1, Vitamin B6, Vitamin D, Vitamin B2, Vitamin K1, Vitamin B12, maltodextrin is used as a carrier |
| Zinc (II) sulfate.7H20 | Zinc sulfate |
| Sodium selenite (IV) 1% | Sodium selenite, maltodextrin is used as a carrier |
| Maganese (II) sulfate | Manganese sulfate |
| Copper (II) sulphate | Copper sulphate |
| Sodium fluoride | Sodium fluoride |
| Chromium (III) chloride | Chromium chloride |
| Sodium molybdate | Sodium molybdate |
| Potassium iodide | Potassium iodide |

### Example 4: Liquid concentrated milk preparation

Skimmed concentrated milk, low in lactose, otherwise known as liquid concentrated milk with a protein content of 11.6g/100g, was added into the overall composition in the amount of 44g/100mL, which equates to 5.1 g/100mL of skimmed concentrated milk protein in the finished product. This amount (5.1 g/100mL) of skimmed milk protein provides about 4.08 g/100mL of micellar casein and 1.02 g/100mL of whey protein.

### Example 5: Powdered milk protein preparation

Dried skimmed milk protein powder with a protein content of 89.5g/100g, was added into the oil phase prior to addition to the overall composition according to the present invention at an amount of 3.75g/100mL, which equates to 3.35 g/100mL of dried skimmed milk protein in the finished product. An amount of 3.35g of dried skimmed milk protein provides 2.68g/100mL of micellar casein and 0.67g/100mL of whey protein.

### Example 6: Dried Soya protein preparation

Dried soya protein with a soya protein content of 90g/100g, was added into the oil phase prior to addition to the overall composition according to the present invention at the amount of 1.5g/100ml, which equates to 1.35 g/100mL in the finished product.

### Example 7: Total protein content in overall composition

The Liquid concentrated milk preparation, powdered milk protein preparation and the dried Soya protein preparation were added into the overall composition to realise a final finished product comprising a combined total of:
- 6.76 g/100mL micellar casein protein derived from the liquid concentrated milk and the powdered milk protein preparations;
- 1.69 g/100mL whey protein derived from the liquid concentrated milk and the powdered milk protein preparations;
- 1.35 g/100ml soy protein derived from the dried soya protein preparation.

A sum total protein content comprising of micellar casein and whey, powdered casein and whey protein and soya protein is provided of 9.8 g/100mL of the overall composition of the finished product. The overall composition of the finished product has a protein factor of 6.38. This represents 9.6g/100mL of protein in the finished product corrected with a protein factor of 6.25 (legal unit for nutritional labelling of food according to regulation 1169/2011.

### Example 8: Method of formulating a nutritional composition according to the present invention

By way of example and not limitation, an example of a process for making the present invention is as follows:
The method of manufacture, generally indicated by reference numeral 100, and described herein, is useful for formulating nutritional compositions according to the present invention.

The method according to the present invention comprises the steps of:
Combining powders of proteins (as prepared according to Example 5 and 6), sugars, minerals, glucose syrup and vitamins and other powdered auxiliary ingredients with the oil phase, herein referred to as rapeseed oil, to make a first mixture 101, wherein the first mixture is free of water.

A second mixture 102 comprises water and concentrated skimmed milk, low in lactose as prepared according to Example 4.

A third mixture 103 comprises flavours, colouring agents, and other suitable auxiliary agents combined with water.

The three mixtures are then filtered 104 using a filter of pore size ~1cm in size and put together in a mixing tank 105 for mixing in a loop and aspiration of powders and liquid by cone. After, hydration and natural degazing 106, the combined mixture undergoes a filtration step 107 using a filter of pore size ~1mm in size, before undergoing sterilization 108 comprising a preheating step, a sterilisation step, a homogenisation step and a cooling step; during the steralisation step 108, the combined mixture undergoes a microbilogical test 109 to determine that sterilisation has been achieved; the combined mixture then undergoes a further filtration step 110 using a filter of pore size ~1mm in size, before being stocked in a buffer sterile tank 111 and incubated for 5 days at 53°C, in preparation for filling 112, under a protective atmosphere into bottles that have been pre-incubated at 30°C for 72hours with a D-count of PE= 0.5 to 1 g, labelling 114 comprising printing and overwrapping, and stocking for quarantine prior to sale 114. Storage (also known as quarantine prior to sale) 114 is for about 12 to 18 months at 4 to 25°C, under relative humidity conditions from 30 to 75% R.H.

A resultant liquid nutritional composition having from 8 to 12 grams of protein per 100mL, from 8 to 12 grams of fat per 100mL, and from 23 to 34 grams of carbohydrates per 100mL, as well as other additional constituents of the liquid nutritional composition, including, but not limited to flavourings, colorants, vitamins, minerals and others is produced.

### Example 9: Synergistic effect of milk proteins and soy protein

A typical synergy study includes a control composition (A) and compositions comprising a protein source derived from: casein protein only (B); whey protein only (C); soy protein only (D); whey plus soy protein (E); and, a combination of casein, whey and soy (F) protein.

The inventors concluded that a combined casein:whey:soy composition of the present invention to provide a relatively high protein concentration (i.e. from 8 to 12 grams per 100mL of composition), that surprisingly retained a shelf stability generally only achieved by lower protein compositions (data not shown).

### Example 10: Patient compliance of nutritional supplement

A clinical trial confirms patient compliance of a nutritional composition such as the composition of the present invention. A typical clinical trial is described below.

In a trial patients ranging from 35 to 65 years old are tested. Half of these patients receiving products with the combined protein mix (casein:whey:soy at a ratio of 2:1:1) and the other half receiving a control composition. The patients tasting the products are invited to rate the product composition with respect to appearance, aroma, taste (overall), and liking of mouthfeel.

The inventors concluded that patient compliance of 95% or above was achievable with patients likely to deem the composition of the present invention palatable and consumable (data not shown).

## Claims

1. A composition with an energy content of at least 100kcal per ml and a pH from 6 to 7.8 comprising:
an overall protein content of at least 13% of the total calories of the overall composition;
a lipid content of at least 30% of the total calories of the overall composition; and,
a carbohydrate content of at least 30% of the total calories of the overall composition;
wherein the overall protein content is formed of a mixture of protein components obtainable from at least two different sources.

2. The composition as claimed in claim 1 wherein, the protein components obtainable from at least two different sources is formed of a combination comprising two or more of micellular milk protein, milk protein concentrate, milk protein isolate and plant protein origin.

3. The composition as claimed in claim 1 wherein, the overall protein content comprises one or more proteins, peptides, amino acids, and precursors or metabolites of said proteins, peptides and amino acids thereof, in an amount that provides at least 13% of the total calories of the composition.

4. The composition as claimed in the preceding claims wherein, the overall protein content is formed of a mixture comprising two or more protein components of:
casein protein;
whey protein; and,
soy protein.

5. The composition as claimed in claim 4 wherein, the casein protein is formed of a combination of casein obtainable from two or more of micellular milk protein, milk protein concentrate, milk protein isolate.

6. The composition as claimed in claim 4 wherein, the whey protein is formed of a combination of whey obtainable from two or more of micellular milk protein, milk protein concentrate, milk protein isolate.

7. The composition as claimed in any of claims 4 to 6 wherein, casein, whey and soy proteins are present in the following amounts:
casein protein is present up to 99% of the total weight of the protein content;
whey protein is present up to 25% of the total weight of the protein content; and,
soy protein is present up to 25% of the total weight of the protein content.

8. The composition as claimed in any of claims 4 to 7 wherein, casein is present up to 50% of the total weight of the protein content.

9. The composition as claimed in claim 7 wherein, the whey protein and the soy protein combined is present up to 25% of the total weight of the protein content.

10. The composition as claimed in the preceding claims wherein, the composition comprises one of:
a probiotic micro-organism or combination of probiotic micro-organisms;
a pharmaceutically active ingredient or combination of pharmaceutically active ingredients; and,
any combination thereof.

11. The composition as claimed in the preceding claims wherein, the composition comprises the addition of at least one auxiliary component of:
fibre;
stabilising agents;
vitamins;
minerals;
emulsifiers;
chelating agents;
masking agents;
bulking agents or fillers;
colourants; and,
flavourings;
or any combination thereof.

12. The composition as claimed in the preceding claims wherein, the composition comprises a water content from 70% to 73% of the total weight of the overall composition

13. A process for making a composition with an energy content of at least 100kcal per mL, a pH from 6 to 7.8 having an overall protein content formed of a mixture of protein components selected from at least two different sources, wherein said process comprises the steps of:
a. combining powdered ingredients, comprising sources of protein, carbohydrate and select auxiliary components comprising vitamins and minerals, or combinations thereof, with a fatty phase to form a first mixture free from water;
b. combining water and a concentrated skimmed milk low in lactose solution to form a second mixture;
c. combining select auxiliary components comprising flavourings and colourants, or combinations thereof, with water to form a third mixture;
d. filtration of the first mixture, the second mixture and the third mixture, using a filter with a pore size of at least 1 cm in size
e. mixing the first mixture, the second mixture and the third mixture together in a mixing tank using a mixing loop, aspiration of powder and liquid by cone to form a final combined mixture;
f. hydratation and natural deglazing of the final combined mixture of step e.
g. filtration of the resultant final combined mixture of step f, using a filter with a pore size of at least 1 mm.
h. continuous tubular UHT sterilisation comprising pre-heating final combined mixture of step g, sterilising the final combined mixture of step g, homogenising the final combined mixture of step g, and cooling the final combined mixture of step g;
i. the combined mixture undergoes a microbilogical test at some point during step h to determine that sterilisation has been achieved;
j. filtration of the resultant final combined mixture of step h, using a filter with a pore size of at least 1 mm.
k. Stocking the resulting final combined mixture of step j in a buffer sterile tank and incubated for 5 days at a temperature from 50 to 60°C prior to filing;
l. filing: 100mL to 150 mL portions are siphoned off from the final combined mixture of step k and transferred to a storage container under a protective atmosphere wherein, the storage container has been pre-incubated at 30°C for 72 hours and with a D-count of PE from 0.5 to 1 g;
m. printing and overwrapping is applied to the storage container filled with the final combined mixture of step I; and,
n. storage of the final combined mixture contained within the storage container as obtained in step I and m at a temperature from 18 to 24°C for up to 18 months prior to consumption.

14. A process for making a composition with an energy content of at least 100kcal per mL, a pH from 6 to 7.8 having an overall protein content formed of a mixture of protein components selected from at least two different sources, as claimed in claim 13, wherein probiotics or pharmaceutical active ingredients are added to the final combined mixture prior to storage.

15. A process for making a composition with an energy content of at least 100kcal per mL, a pH from 6 to 7.8 having an overall protein content formed of a mixture of protein components selected from at least two different sources, as claimed in claim 13 or 14 wherein the first mixture partially provides the protein components that make up the overall protein content of the overall composition.
